# EUROPÄISCHE PATENTSCHRIFT

(11) **EP 1 225 447 B1**
(45) Veröffentlichungstag und Bekanntmachung des Hinweises auf die Patenterteilung: **26.11.2008**
(21) Anmeldenummer: 02000005.5
(22) Anmeldetag: 02.01.2002
(51) Int. Cl.: G01N 33/18, G01N 31/00, G01N 31/12

(54) **Katalysator zur TC- und TNb- Messung bei Wasserproben sowie dessen Verwendung**
Catalyser for TC and TNb measurement in water samples and use thereof.
Catalyseur pour la mesure des valeurs TC et TNb d'échantillons d'eau et utilisation.

(30) Priorität: 05.01.2001 DE 10100259
(43) Veröffentlichungstag der Anmeldung: 24.07.2002
(73) Patentinhaber: DIMATEC Analysentechnik GmbH, 45141 Essen (DE)
(72) Erfinder: Dirsus, Walter, 45276 Essen (DE); Monsé, Christian, Dr., 44801 Bochum (DE); König, Julius, 44801 Bochum (DE)
(74) Vertreter: Kreutzer, Ulrich

(56) Entgegenhaltungen:
- EP-A- 0 075 467
- WO-A-94/07134
- FR-A- 2 408 139
- DATABASE WPI Section Ch, Week 199644 Derwent Publications Ltd., London, GB; Class H06,Page 05, AN 1996-441287 XP002259071 & RU 2 053 019 C (KOROBSKOI BS), 27. Januar 1996 (1996-01-27)
- DATABASE WPI Section Ch, Week 199824 Derwent Publications Ltd., London, GB; Class E36,Page 10, AN 1998-270041 XP002259072 & RU 2 093 249 C (AS SIBE CATALYSIS INST), 20. Oktober 1997 (1997-10-20)
- PATENT ABSTRACTS OF JAPAN vol. 1997, no. 10, 31. Oktober 1997 (1997-10-31) & JP 09 155194 A (RIKEN CORP), 17. Juni 1997 (1997-06-17)
- DATABASE WPI Section Ch, Week 197925 Derwent Publications Ltd., London, GB; Class D15, AN 1979-46068B XP002259073 & JP 54 056893 A (SUMITOMO CHEM. CO. LTD), 8. Mai 1979 (1979-05-08)

## Beschreibung

Die Erfindung betrifft einen Katalysator für den Einsatz bei der Bestimmung von TC- und TN_{b}-Werten von Wasserproben sowie ein Verfahren zur Herstellung des in von außen beheizten Reaktoren im heißen Gasstrom einsetzbaren Katalysators.

Die TN_{b}- und TC-Werte sind für die Überwachung der Wassergüte von hoher Aussagekraft. Dabei sind sie ebenfalls sehr wichtig für den laufenden Betrieb einer Kläranlage, weil auf diese Art und Weise die Belastungen frühzeitig berücksichtigt und die gesamte Anlage darauf eingestellt werden kann. Aus der WO 94/07134 A1 ist ein Verfahren bekannt, bei dem Wasserproben bei Temperaturen über 700 °C im Sauerstoffstrom verbrannt werden. Die Gase werden durch die Apparatur geleitet, wobei in einem dem Reaktor nachgeordneten Kondensatabscheider das Gas vom größten Teil der Feuchtigkeit befreit wird. Es ist auch möglich Trockner gesondert vorzusehen, um die Restfeuchte wegzunehmen. Dieser bekannte Stand der Technik sieht allerdings nur die Ermittlung von Stickstoff vor, wobei zunächst bei der Verbrennung Stickstoffmonoxid entsteht. Liegt hierzu im Verhältnis ein nicht zu vernachlässigender Anteil sauerstoffreicher Stickoxide vor, werden diese in einem NOₓ-NO-Konverter in Stickstoffmonoxid überführt. Unter Zugabe von Ozon wird das Stickstoffmonoxid in Stickstoffdioxid überführt, bevor es einem Chemolumineszenz-Detektor passiert. Die hier ermittelte Strahlungsenergie ist der ursprünglichen NO-Konzentration proportional und gestattet somit dessen quantitative Bestimmung. Ähnlich ist das Verfahren nach der EP 0 075 467 A1, wobei verschiedene Katalysatoren wie auch Platin eingesetzt werden. Die DE-OS 42 31 620 beschreibt eine Vorrichtung, die den Gesamtgehalt an organischen Kohlenstoff und an Stickstoff im Wasser misst. Die Messung erfolgt in einem Gasanalysator, der zwei doppelseitige Küvetten mit jeweils einer Hälfe für das Messgas und einer Hälfte für ein Vergleichsgas besitzt. Über Zugabe modulierter Lichtstrahlen werden Kammern durchstrahlt, die jeweils mit einer der zu bestimmenden Komponenten der Gasprobe gefüllt sind, der eine Raum mit CO₂, der andere mit NO. Die entsprechende Vorrichtung hat sich als sehr aufwendig und zudem störanfällig gezeigt, so dass für den TN_{b}-Anteil im Wesentlichen die Chemolumineszenz-Methode eingesetzt wird, während CO₂ nach Abscheidung des Wassers IRspektroskopisch bestimmt wird. Die Chemolumineszenz-Methode ist eine vergleichende Methode, d. h. Der TC- bzw. Der TN_{b}-Gehalt einer Probe wird mittels einer Kalibrierkurve ermittelt. Die Kalibrierung nach DIN erfolgt für die Kohlenstoffbestimmung mit einem Standard, bestehend aus Kaliumhydrogenphthalat (KHP) und für die Stickstoffbestimmung aus einem Mischstandard bestehend aus 50 % Kaliumnitrat (KNO₃) und 50 % Ammoniumsulfat ((NH₄)₂SO₄). Bei dem Verbrennungsprozess nach diesem Verfahren wird der gebundene Stickstoff je nach Substanzklasse unterschiedlich gut zu Stickoxiden umgesetzt. Bei einigen Verbindungen werden erhebliche Stickstoffminderbefunde festgestellt, da bei der thermischen Zersetzung mehr oder weniger elementarer Stickstoff (N₂) freigesetzt wird, der sich diesem Detektionsverfahren entzieht. Somit ist es erforderlich, für eine problematische Stoffklasse eine gesonderte Kalibrierung durchzuführen. Dies ist sehr aufwendig und kann das ganze Verfahren schon von den Kosten her unattraktiv machen. Für die Spaltung de Abgase werden in Database WPI Section CU. Derwent abstract 1996 - 441287 und auch 19998 - 270 041 Katalysatoren eingesetzt, bei dem auf Basaltfäden, z. B. Cobalt- und Chromnitrate aufgebracht sind. Auch bei dem Verfahren nach der FR - A 2 408 139 wird u. a. mit einem Platinkatalysator gearbeitet, der nachweislich nur geringe Wiederfindungsraten aufweist.

Der Erfindung liegt daher die Aufgabe zu Grunde, einen Katalysator für die Bestimmung der TC- und TB_{b}-Werte von Wasserproben mit hohem Wiederfindungsraten zu entwickeln und ein Herstellungsverfahren dafür zu schaffen.

Die Aufgabe wird gemäß der Erfindung dadurch gelöst, dass ein als Trägermaterial dienender körniger Bimsstein eingesetzt ist, auf dessen Oberfläche Cobalt(II/III)-oxide und Chrom(III)-oxide als Gemisch aufgebracht sind. Dieses Aufbringen der Oxide erfolgt über einen Brenn- bzw. Glühvorgang, mit ∼ 1000 °C Die entsprechenden Katalysatorkörner oder kugelförmigen Teilchen werden im Verbrennungsreaktor zwischen zwei Glaswolleschichten angeordnet und können so ihre Aufgabe vorteilhaft wahrnehmen, nämlich die Beeinflussung und Umwandlung von Gasen in CO₂ und in verschiedene Endverbindungen , also Stickstoffverbindungen, deren Konzentration sicher mit Hilfe einer einfachen Kalibrierung dann möglich wird.

Mit Hilfe dieser besonderen Katalysatoren ist es zunächst einmal möglich, die TC- und TN_{b}-Werte mit Hilfe der Kalibriermethode bzw. der Kalibrierkurven zu bestimmen, ohne dass die weiter oben beim Stand der Technik beschriebenen negativen Eigenschaften auftreten, nämlich ohne dass irgendwelche Stickstoffminderbefunde festzustellen sind. Vielmehr ergibt sich ein insgesamt und durchgängiges gleichmäßiges Aufschlüsseln der entsprechenden Werte, wobei für alle Stoffklassen, auch für die recht problematischen Stoffklassen, eine gesonderte Kalibrierung nicht durchgeführt werden muss. Es kann vielmehr mit einer Kalibrierung gearbeitet bzw. Einer Kalibrierkurve gearbeitet werden, was für die Gesamtauswertung erhebliche Vorteile mit sich bringt. Dafür verantwortlich ist vor allem der gezielt aufgebaute Katalysator. Vorteilhaft ist dabei, dass nicht nur bei der TN_{b}-Bestimmung die Wiederfindungsraten unterschiedlicher Substanzen gleich bleibend sind, sondern auch bei der TC-Bestimmung. Damit lässt sich ein insgesamt optimales und betriebssicheres Bestimmungsverfahren der TC- und TN_{b}-Werte verwirklichen, dies insbesondere auch deshalb, weil der zum Einsatz kommende Katalysator Kohlenstoffverbindungen quantitativ zu CO₂ umgesetzt, so dass sie ebenfalls leicht mit bekannter Methode auszuwerten sind.

Das Trägermaterial aus Bimsstein ist besonders geeignet, wenn es eine Körnung 0,5 - 5,0 mm, vorzugsweise 0,8 - 3,0 mm aufweist. Eine solche Körnung lässt sich auch vorteilhaft gut im Verbrennungsreaktor verarbeiten, gibt aber wegen der großen Oberfläche, vor allem bei späterem Einsatz, die notwendige Betriebssicherheit und zwar im Prinzip auch dann, wenn die Bedienungsmannschaft nicht wie vorgeschrieben sorgfältig arbeitet.

Um eine besonders gleichmäßige Beschichtung der einzelnen Trägermaterialkörner zu gewährleisten, ist vorgesehen, dass die Cobalt(II/III)-oxide und Chrom(III)-oxide als Gemisch auf das Trägermaterial in zwei getrennten Beschichtungsvorgängen aufgebrannt sind, da die im ersten Beschichtungsvorgang eventuell nicht gleichmäßige Beschichtungsdicke durch den zweiten Beschichtungsvorgang ausgeglichen werden kann. Damit ist sichergestellt, dass rundum eine ausreichende Beschichtungsdicke vorhanden ist, die die nachfolgenden Einsätze im Verbrennungsreaktor möglich machen.

Weiter oben ist erläutert worden, dass der Katalysator das für die TN_{b}- und TC-Messwerte wichtige Material in einer Doppelbeschichtung aufgetragen wird, wobei gemäß der Erfindung jede der beiden Schichten auf dem Trägermaterial aus einem Gemisch von Cobaltnitrat, Ammoniumdichromat und Wasser besteht, das nach dem Trocknen unter Umwandlung in Cobalt(II/III)-oxid und Chrom(III)-oxid aufgebrannt ist. Wichtig ist also, dass bei beiden Beschichtungsvorgängen das gleiche Gemisch verwendet wird, um die Gleichmäßigkeit der Beschichtung zu gewährleisten und ein immer sicheres Betriebsergebnis.

Der weiter vorn beschriebene Katalysator wird auf eine bestimmte Art und Weise, d. h. nach einem bestimmten Verfahren hergestellt, was die Sicherheit gibt, dass ein immer gleicher Katalysator zur Verfügung gestellt wird. Dieses Verfahren zeichnet sich dadurch aus, dass ein Trägermaterial aus Bimsstein mit einer Mischung aus Cobaltnitrat (Co(NO₃)₂ x 6H₂O), Ammoniumdichromat (NH₄)₂Cr₂O₇) und Wasser versetzt, nach Abfiltern der überschüssigen Mischung getrocknet und anschließend bei 1000 °C geglüht wird. Die beschriebene Mischung kann auf einfache und sichere Art und Weise auf das poröse Trägermaterial, also den Bimsstein aufgebracht werden, um dann durch Trocknen und Einbrennen dessen Oberfläche zu verdecken. Der Bimsstein mit seiner rauen Oberfläche sorgt dann dafür, dass die entstehenden Oxide sicher und fest anhaften.

Um sicherzustellen, dass die enthaltene Katalysatorkörnermenge eine immer sichere und wirksame Beschichtung aufweist, ist vorgesehen, dass der Beschichtungsvorgang einschließlich Glühvorgang mehrfach, vorzugsweise ein zweites Mal wiederholt wird. Über diesen Doppelbeschichtungsvorgang ist eine Rundumbeschichtung sichergestellt, gleichzeitig aber erreicht, dass der Herstellungsprozess finanziell in Grenzen bleibt, da eine konkrete Nachprüfung der einzelnen Körner des Katalysators überflüssig sind.

Das Oxidieren bzw. das Glühen der die Beschichtung des Katalysators ergebenden Komponenten wird dadurch optimiert, dass das Trägermaterial mit der Beschichtung bei 125 °C getrocknet und anschließend für∼ 2 Stunden bei ∼ 1000 °C geglüht wird. Durch die vorherige Trocknung des Trägermaterials wird verhindert, dass Wasserdampf innerhalb der Beschichtung eingeschlossen wird. Durch diese Verfahrensführung bleibt sichergestellt, dass sich die Oberfläche des entsprechenden Kornes des Katalysators als durchgehend und glatt erweist.

Die entsprechend zu behandelnde Mischung und auch auf das Trägermaterial aufzubringende Mischung besteht gemäß der Erfindung aus 10,0 g Cobaltnitrat(Co(NO₃)₂ x 6H₂O) (34,4 mmol), 2,0 g Ammoniumdichromat (NH₄)₂Cr₂O₇) (7,9 mmol) und 8,0 g Wasser, die mit ∼ 7,0 g Bimsstein mit einer Körnung von 0,8 - 3,0 mm vermischt wird. Wie schon erwähnt, ist es so möglich, schon beim eigentlichen Beschichtungsvorgang, also dem Mischen des Trägermaterials, d. h. des Bimssteins und der Mischung eine optimale Beschichtung auf der Oberfläche des Bimssteins vorzugeben, so dass dann beim nachfolgenden Glühen auch wirklich eine durchgehende und ausreichend dicke Schicht auf dem Trägermaterial vorhanden ist.

Gemäß Herstellungsverfahren des Katalysators ist es denkbar und vorteilhaft, das Gemisch aus Flüssigkeit und Trägermaterial ein- oder mehrfach zu evakuieren. Durch kurzzeitiges Evakuieren wird erreicht, dass die Flüssigkeit bzw. die Mischung in das Trägermaterial eindringt bzw. dieses durchdringt, so dass die schon erwähnte gewünscht optimal abgesicherte Oberfläche erreicht wird.

Die Erfindung zeichnet sich insbesondere dadurch aus, dass ein Katalysator sowie ein Herstellungsverfahren vorgesehen ist, mit dem ein neuer Katalysator in dem Bereich TC- und TN_{b}-Messunbg bzw. -Bestimmung eingesetzt werden kann, der optimale Ergebnisse ermöglicht. Insbesondere ist es mit dem beschriebenen Katalysator möglich, die Bildung von Stickoxiden je nach Substanzklasse gleich bleibend zu belassen bzw. zu erhalten, wodurch sichergestellt ist, dass die Wiederfindungsraten unterschiedlicher Substanzen nahezu identisch sind. Diese Eigenschaft ist erwünscht, da zur Konzentrationsbestimmung verschiedener N-Verbindungen nur die DIN-konforme Kalibrierung ausreicht. Zusätzlich setzt der neue Katalysator Kohlenstoffverbindungen quantitativ zu CO₂ um, so dass eine simultane Bestimmung der Parameter TC und TN_{b} erreicht wird. Es hat sich gezeigt, dass für die TC-Messbereiche zwischen ca. 10 - 750 mg/l Kohlenstoff und TN_{b}-Messbereiche zwischen 1-100 mg/Stickstoff erfreulich genaue Ergebnisse zu erreichen sind. Beispiele dazu weiter hinten aufgeführt.

Weitere Vorteile und Einzelheiten des Erfindungsgegenstandes ergeben sich aus der nachfolgenden Beschreibung der zugehörigen Zeichnungen, in der ein bevorzugtes Ausführungsbeispiel dargestellt ist. Dabei zeigen:
- Fig. 1: einen Verbrennungsreaktor in Seitenansicht,
- Fig. 2: ein Katalysatorkorn im Schnitt in einschaliger Ausführung,
- Fig. 3: das Katalysatorkorn in zweischaliger Ausführungs-Form und
- Fig. 4: eine schematische Wiedergabe der Versuchsanordnung zur Ermittlung der TC- und TN_{b}-Werte von Wasserproben.

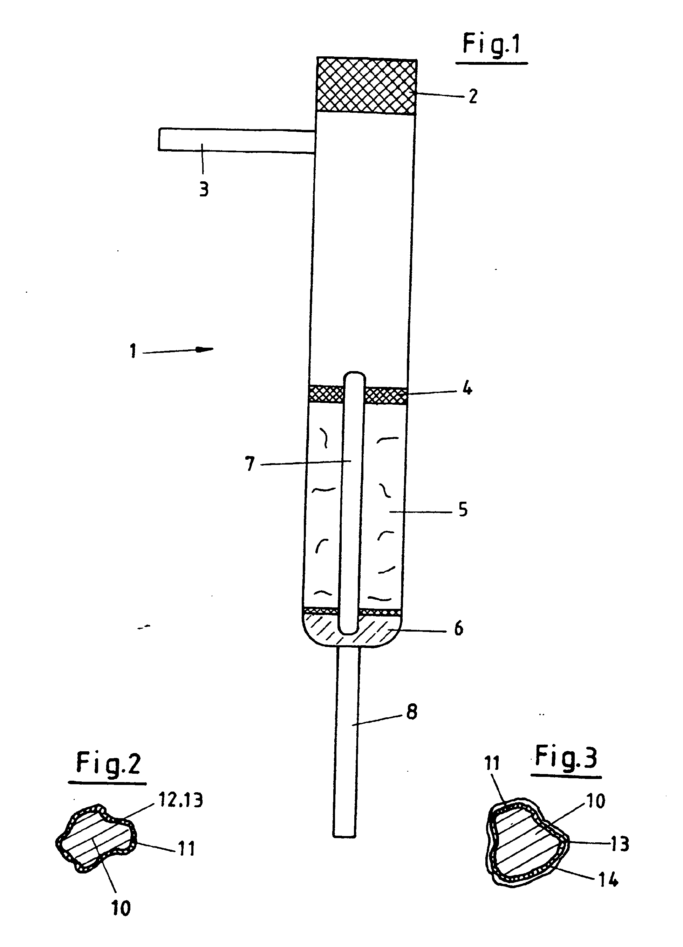

Fig. 1 zeigt den Verbrennungsreaktor 1 in Seitenansicht. Oben ist ein Verschlussstopfen 2 vorgesehen, während der Gaseinlass für den Sauerstoff mit 3 bezeichnet ist. Bei dem Verbrennungsreaktor 1 handelt es sich um einen Reaktor, der sowohl für den Versuchsbetrieb wie auch für den Normalbetrieb geeignet ist. Im Reaktorinnenraum ist der weiter hinten noch näher erläuterte Katalysator zwischen einer unteren Quarzwollschicht 6 und einer oberen Quarzwollschicht 4 sicher angeordnet. Durch die eingeklemmte Bauweise wird ein mechanischer Antrieb beim Einspritzen der Probe oder auch beim Durchspülen mit anderen Gasen verhindert, zumal die Entzündung fast explosionsartig erfolgt.

Im Bereich des Katalysators 5 ist eine Einbuchtung 7 erkennbar, in der ein Thermoelement angeordnet werden kann, um den Verbrennungsprozess genau überwachen zu können. Das den Katalysator 5 durchströmende Gas, das aus der Verbrennung von Sauerstoff und der Wasserprobe resultiert, verlässt den Verbrennungsreaktor 1 im Bereich des Reaktorfußes 8.

Figuren 2 und 3 zeigen in vergrößerter Wiedergabe das Trägermaterial 10, hier in Form von 0,8 - 3,0 mm großen Bimssteinkörnern, deren Oberfläche 10 mit einer Oxidbeschichtung 12 versehen ist. Entsprechendes verdeutlicht Fig. 2. Bei der Ausführung nach Fig. 3 kommt eine zweite Schicht 14 zur ersten Schicht 13 hinzu, wobei beide zusammen die Oxidbeschichtung 12 ergeben, die rundum möglichst das Korn des Trägermaterials 10 umhüllt.

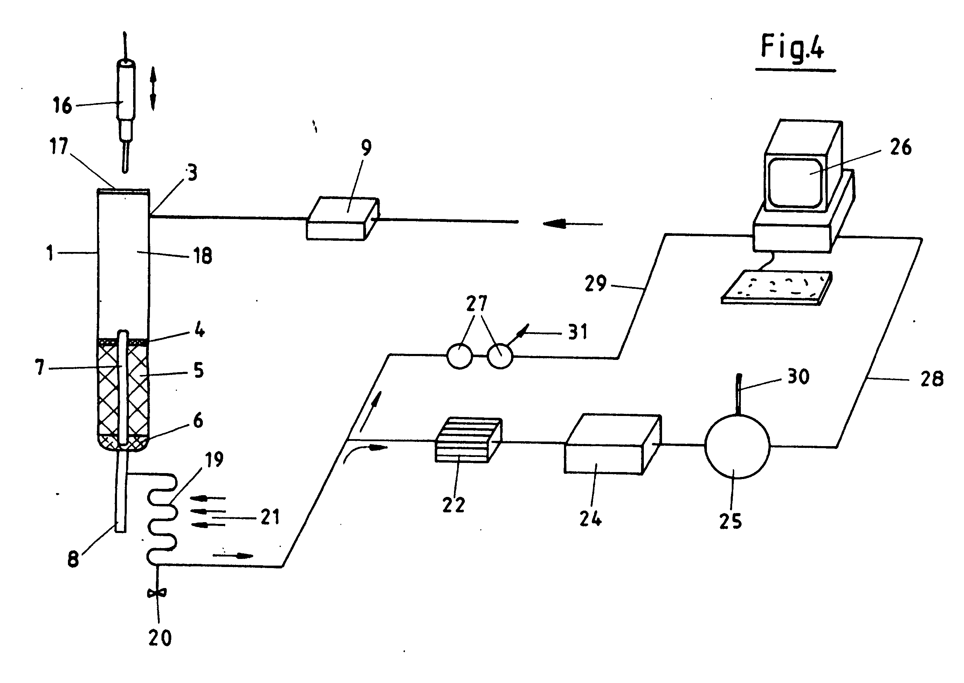

Fig. 4 zeigt eine Versuchsanordnung, bei der der Sauerstoff über den Mengenregler 9 in das Glasgefäß 18 hineingeleitet wird. Die einzelne Wasserprobe wird über den Dosierer 16 durch die Dichtmembran 17 hindurch in den Innenraum des Glasgefäßes 18 eingesprüht. Durch gleichzeitiges Einleiten von Sauerstoff verbrennt diese Probe kurzfristig und die Verbrennungsgase können dann weiter durch die Quarzwolleschicht 4 und 6 hindurchgezogen werden, wobei zwangsweise die Gase den Katalysator 5 durchströmen, wobei der Katalysator 5 dafür sorgt, dass die Kohlenstoffverbindungen quantitativ zu CO₂ umgesetzt werden, während die Stickoxide unabhängig von der Substanzklasse gleichbleibend gebildet werden, so dass anschließend die Chemolumineszenz-Methode angewendet werden kann.

Die Gase durchströmen nach Verlassen des Reaktorfußes 8 einen Abscheider für Kondensat 19 mit einem Kondensatauslass 20 und einer Kühlung 21, um auf diese Art und Weise die Gase möglichst weit abzutrocknen. Der danach vorgesehene Nachtrockner 22 kann vor der Teilung der Leitung angeordnet werden oder wie gezeigt im Bereich des nachgeordneten Konverters 24, wo die Stickoxide zu Stickstoffmonoxid umgewandelt werden.

Das Chemolumineszenzgerät ist mit 25 bezeichnet, wobei die Gase über den Gasauslass 30 abströmen können, während über die Datenleitung 28 die entsprechenden Informationen direkt der Datenverarbeitung 26 zugeleitet werden können.

Mit 27 ist ein IR-Spektrograph bezeichnet und mit 31 der Auslass, wo das CO₂ die Apparatur verlässt, während die entsprechenden Daten über die Datenleitung 29 ebenfalls der Datenverarbeitung 26 zugeführt werden, um hier schnell und sicher ausgewertet zu werden.

Nachfolgende Beispiele verdeutlichen, in welchem Bereich und mir welch hoher Wiederfindungsrate das erfindungsgemäße Verfahren und mit dem erfindungsgemäßen Katalysator betrieben werden kann. Mit TN_{b}-Mischstandard und TC-Standard, d. h. mit den beiden ersten Tabellen sind die ermittelten Standardwerte wiedergegeben, während die weiteren Tabellen die hohe Wiederfindungsrate verdeutlichen.

### Wiederfindungsraten:

**TN_{b}-Mischstandard:**

| | **30.11** | **1.12** | **1.12** | **2.12** | **5.12.** | **6.12** | **7.12** | **8.12** | **13.12** | **∅** | **+/- (%)** | **∅ (%)** |
|---|---|---|---|---|---|---|---|---|---|---|---|---|
| **100 mg/l N:** | 102,8 | | 105,2 | 101,5 | 109,7* | 111,6* | 88,4* | 83,1* | 94,7 | **101,1** | **1,7** | **101,1 %** |
| **75 mg/l N:** | 75,1 | | 76,5 | 73,2 | 77,5 | 80,0* | 74.8 | 76,1 | 71,3 | **74,9** | **0,7** | **99,9 %** |
| **50 mg/l N:** | 49,7 | | 50,1 | 48,0 | 50,1 | 51,6 | 49,7 | 50,4 | 49,0 | **49,8** | **0,9** | **99,6 %** |
| **25 mg/l N:** | 24,3 | 24,2 | 24,1 | 24,1 | 24,9 | 24,3 | 25,1 | 25,6 | 24,0 | **24,5** | **0,7** | **98,0 %** |
| **10 mg/l N:** | 9,6 | 9,7 | 9,7 | 9,7 | 10,0 | 9,8 | 9,7 | 9,8 | 9,8 | **9,8** | **0,7** | **98,0 %** |
| **7,5 mg N:** | | 7,2 | 7,3 | 7,1 | 7,6 | 7,2 | 7,2 | | | **7,3** | **1,5** | **97,3 %** |
| **5 mg/l N:** | 4,9 | 4,7 | 4,8 | 4,4 | 4,9 | 4,6 | 4,7 | | | **4,7** | **1,2** | **94,0 %** |
| **2,5 mg/l N:** | 2,4 | 2,3 | 2,3 | 2,2 | 2,3 | 2,1 | 2,2 | | 2,4 | **2,3** | **1,3** | **92,0 %** |
| **1 mg/l N:** | 1,1 | 0,9 | 0,9 | 8,9 | 0,9 | 0,8 | 0,8 | | 1,1 | **0,9** | **1,2** | **90,0 %** |

| | | | | | | | | | | | | |
|---|---|---|---|---|---|---|---|---|---|---|---|---|
| Durchschmittliche Wiederfindung (1 - 100 mg/l): 97 % | | | | | | | | | | | | |

**TC-Standard:**

| | **30.11** | **1.12** | **1.12** | **2.12** | **5.12** | **5.12** | **6,12** | **7.12** | **13.12** | **+/- (%)** | **∅** | **∅ (%)** |
|---|---|---|---|---|---|---|---|---|---|---|---|---|
| **750 mg/l C:** | | | | | | | | 766,7 | | **0,7** | **766,7** | **102,2 %** |
| **500 mg/l C:** | | | | | | | | 503,3 | | **1,0** | **503,8** | **100,8 %** |
| **400 mg/l C:** | | | | | | | | 398,1 | | **1,1** | **398,1** | **99,5 %** |
| **300 mg/l C:** | | | | | | | | 301,8 | | **0,9** | **301,8** | **100,6 %** |
| **200 mg/l C:** | | | | | | | | 205,8 | | **0,2** | **205,8** | **102,9 %** |
| **100 mg/l C:** | 98,3 | 99,0 | 97,3 | 95,1 | 99,5 | | 99,7 | 99,2 | 97,7 | **0,9** | **98,2** | **98,2 %** |
| **75 mg/C:** | 73,6 | 73,6 | 72,1 | 71,2 | 72,9 | | 74,0 | 74,4 | 74,4 | **0,8** | **73,3** | **97,7 %** |
| **50 mg/l C:** | 49,3 | 49,6 | 47,5 | 47,5 | 49,1 | 49,2 | | 49,5 | 50,8 | **1,3** | **49,1** | **98,2 %** |
| **25 mg/l C:** | 25,4 | 24,9 | 24,0 | 24,4 | 24,9 | 25,2 | 25,1 | 24,7 | 25,1 | **1,2** | **24,9** | **99,6 %** |
| **10 mg/l C:** | 9,7 | 9,6 | 9,4 | 9,2 | 9,8 | 9,4 | 9,5 | 9,7 | 9,7 | **1,5** | **9,6** | **96,0 %** |
| **7,5 mg/l C:** | | | | | 7,8 | | 7,6 | | | **0,3** | **7,7** | **102,7 %** |
| **5 mg/l C:** | | | | | 5,0 | | 5,1 | | | **1,5** | **5.1** | **102,0 %** |
| **2,5 mg/l C:** | | | | | 2.3 | | 2,6 | | | **11,6** | **2,5** | **100,0 %** |
| **1 mg/l C:** | | | | | 0,9 | | 1,1 | | | **10,6** | **1,0** | **100,0 %** |

| | | | | | | | | | | | | |
|---|---|---|---|---|---|---|---|---|---|---|---|---|
| Durchschnittliche Wiederfindung bei C (1 - 750 mg/l): 100 % | | | | | | | | | | | | |

**Kaliumnitrat:**

| | **30.11** | **1.12** | **1.12** | **2.12** | **5.12** | **7.12** | **8.12** | **13.12** | **Ø** | **Ø (%)** |
|---|---|---|---|---|---|---|---|---|---|---|
| **100 mg/l N:** | 109,0 | 115,0 | 112,7 | 112,3 | 113,3 | 98,3 | 98.4 | 110,1 | **108,6** | **108,6 %** |
| **50 mg/l N:** | 50,0 | 51,7 | 50,9 | 50,7 | 52,8 | 50,3 | 50,7 | 51,5 | **51,0** | **102,0 %** |
| **10 mg/l N:** | 9,2 | 9,4 | 9,2 | 9,1 | 9,8 | 9,7 | 9,7 | 9,9 | **9,5** | **95,0 %** |
| **2,5 mg/l N:** | 2,3 | 2,2 | 2,2 | 2,1 | 2,2 | 2,2 | 2,2 | **2,4** | **2,2** | **88,0 %** |

| | | | | | | | | | | |
|---|---|---|---|---|---|---|---|---|---|---|
| Durchschnittliche Wiederfindung (2,5 - 100 mg/l): 98 % | | | | | | | | | | |

**Ammoniumsulfat:**

| | **30.11** | **1.12** | **1.12** | **2.12** | **5.12** | **5.12** | **6.12** | **7.12** | **13.12** | **Ø** | **Ø (%)** |
|---|---|---|---|---|---|---|---|---|---|---|---|
| **100 mg/l N**: | 99,2 | 100,2 | 102,1 | 102,4 | 99,2 | 98,2 | 97,6 | 89,5* | 97,3 | **99,5** | **99,5 %** |
| **50 mg/l N:** | 48,5 | 49,9 | 49,4 | 49,6 | 49,1 | 48,7 | 49,6 | 47,3* | 48,2 | **49,1** | **98,3 %** |
| **10 mg/l N:** | 9,5* | 9,9 | 9,9 | 9,9 | **9,8** | 9,9 | 9,9 | 9,8 | 9,7 | **9,9** | **99,0 %** |
| **2,5 mg/l N:** | 2,5 | 2,5 | 2,5 | 2,5 | 2,5 | 2,5 | 2,5 | 2,4 | 2,7 | **2,5** | **100,0** |

| | | | | | | | | | | | |
|---|---|---|---|---|---|---|---|---|---|---|---|
| Durchschnittliche Wiederfindung (2,5 - 100 mg/l): 99 % | | | | | | | | | | | |

**Harnstoff:**

| | **30.11** | **1.12** | **1.12** | **2.12** | **5.12** | **5.12** | **6.12** | **7.12** | **8.12** | **13.12** | **Ø** | **Ø (%)** |
|---|---|---|---|---|---|---|---|---|---|---|---|---|
| **100 mg/l N:** | 104,8 | 104,8 | 106,8 | 104,2 | 110,0* | 105,3 | 106,3 | 92,2* | 93,6* | 103,7 | **105,1** | **105,1 %** |
| **50 mg/l N:** | 50,6 | 50,1 | 51,1 | 50,3 | 52,0 | 51,1 | 51,4 | 48,1 | 49,4 | 51,2 | **50,5** | **101,1 %** |
| **10 mg/l N:** | 9,9 | 9,8 | 10,0 | 9,8 | 9,9 | 10,0 | 10,0 | 9,8 | 10,0 | 9,8 | **9,9** | **99,0 %** |
| **2,5 mg/l N:** | 2,6 | 2,5 | 2,5 | 2,5 | 2,5 | 2,5 | 2,5 | 2,5 | 2,4 | 2,6 | **2,5** | **100 %** |
| **42,9 mg/l C:** | 44,6 | 45,0 | 43,5 | 44,4 | 45,3 | 44,8 | 45,5 | 43,9 | 45,3 | 45,7 | **44,8** | **104,4 %** |
| **21,5 mg/l C:** | 22,6 | 22,3 | 21,7 | 22,1 | 22,5 | 22,4 | 23,0 | 22,6 | 22,9 | 22,8 | **22,5** | **104,7 %** |
| **4,3 mg/C:** | 6,4 | 5,3 | 5,3 | 5,2 | 5,0 | 4,7 | 4,6 | 5,1 | 4,8 | 5,0 | **5,2** | **120,9 %** |
| **1,1 mg/l C:** | 0,8* | 0,0* | 3,2 | 0,0* | 1,3 | 1,0 | 1,0 | 1,0 | 1,0 | **1,4** | **1,6** | **145,5 %** |

| | | | | | | | | | | | | |
|---|---|---|---|---|---|---|---|---|---|---|---|---|
| Durchschnittliche Wiederfindung bei N (2,5 -100 mg/l): 101 % Durchschnittliche Wiederfindung bei C (50 -100 mg/l N): 104 % | | | | | | | | | | | | |

**Glycin:**

| | **30.11** | **1.12** | **5.12** | **6,12** | **7.12** | **13.12** | **Ø** | **Ø (%)** |
|---|---|---|---|---|---|---|---|---|
| **100 mg/l N:** | 95,0 | 95,7 | 97,2 | 96,4 | 88,1* | **95,4** | **95,9** | **95,9 %** |
| **50 mg/l N:** | 47,1 | 47,6 | 48,2 | 48,4 | 47,4 | 48,3 | **47,8** | **95,6%** |
| **10 mg/l N:** | 9,5 | 9,7 | 9,8 | 9,7 | 9,8 | 9,7 | **9,7** | **97,0%** |
| **2,5 mg/l N:** | 2,4 | 2,4 | 2,5 | 2,4 | 2,4 | 2,7 | **2,4** | **96,0%** |
| **171,5 mg/l C:** | 170,8 | 168,9 | 175,0 | 175,7 | 177.5 | 180,9 | **172,5** | **100,6 %** |
| **85,8 mg/l C:** | 84,3 | 84,1 | 87,5 | 88,1 | 86,0 | **88,7** | **86,5** | **100,8 %** |
| **17,2 mg/C:** | 17,4 | 17,4 | 17,6 | 17,7 | 17,4 | 17,9 | **17,6** | **102,3 %** |
| **4,3 mg/l C:** | 5,2 | 5,0 | 4,7 | 4,8 | 4,6 | 4,9 | **4,9** | **114,0 % *** |

| | | | | | | | | |
|---|---|---|---|---|---|---|---|---|
| Durclischnittliche Wriederfindung bei N (2,5 - 100 mg/I): 96 % Durchschnittliche Wiederfindung bei C (10 - 104 mg/l N): 100 % | | | | | | | | |

**Nicotinsäure:**

| | **30.11** | **1.12** | **5.12** | **6.12** | **8.12** | **13.12** | **Ø** | **Ø (%)** |
|---|---|---|---|---|---|---|---|---|
| **100 mg/l N:** | 101,6 | 97,7 | 101,0 | 99,2 | 91,9* | 100.7 | **100,0** | **100,0 %** |
| **50 mg/l N:** | 47,7 | 47,6 | 48,7 | 47,5 | 48,0 | | **47,9** | **95,8 %** |
| **10 mg/l N:** | 9,5 | 9,6 | 9,7 | 9,6 | 9,6 | 9,7 | **9,6** | **96,0 %** |
| **2,5 mg/l N:** | 2,4 | 2,4 | 2,5 | 2,4 | 2,4 | | **2,4** | **96,0 %** |
| **514,5 mg/l C:** | 486,2* | 516.2 | 529,5 | 532,2 | 533,6 | 506,8 | **523,7** | **101,8 %** |
| **257,3 mg/l C:** | 241,3 | 252,0 | 257,5 | 258,5 | 266,8 | | **255,2** | **99,2 %** |
| **51,5 mg/C:** | 51,7 | 51,4 | 51,8 | 52,4 | 53,2 | 41,5* | **52,1** | **101,2 %** |
| **12,9 mg/l C:** | 12,5 | 13,2 | 12,5 | 12,9 | 12,9 | | **12,8** | **99,2 %** |

| | | | | | | | | |
|---|---|---|---|---|---|---|---|---|
| Durchschnittliche Wiederfindung bei N (2,5 -100 mg/l): 97 % Durchschnittliche Wiederfindung bei C (2,5 - 100 mg/l N): 100 % | | | | | | | | |

Bei den nachfolgenden Substanzen tritt das Problem der schnellen Alterung der angesetzten Maßlösungen auf. Daher sind die Wiederfindungsraten im C. und N-Bereich bei frisch angesetzten Lösungen besser.

### EDTA:

| | **30.11** | **1.12** | **2.12** | **5.12** | **6,12** | **Ø** | **Ø (%)** |
|---|---|---|---|---|---|---|---|
| **100 mg/l N:** | 89,3 | 87,7 | 83,8 | 90,1 | 88,7 | **87,9** | **87,9 %** |
| **50 mg/l N:** | 45,3 | 43,8 | 41,8 | 43,6 | 43,3 | **43,6** | **87,2 %** |
| **10 mg/l N:** | 9,3 | 9,3 | 8,9 | 9,4 | 9,2 | **9,2** | **92,0 %** |
| **2,5 mg/l N:** | 2,4 | 2,4 | 2,3 | 2,4 | 2,4 | **2,4** | **96,0 %** |
| **428,7 mg/l C:** | 418,2 | 439,2 | 410,2 | 443,3 | 445,7 | **431,3** | **100,6 %** |
| **21,4 mg/l C:** | 207,5 | 213,3 | 206,1 | 217,9 | 218,8 | **211,5** | **98,6 %** |
| **42,9 mg/C:** | 42,5 | 42,8 | 41,4 | 43,6 | 44,3 | **42,9** | **100,0 %** |
| **10,7 mg/l C:** | 9,9 | 10,6 | 9,5 | 10,9 | 10,8 | **10,3** | **96,3 %** |

| | | | | | | | |
|---|---|---|---|---|---|---|---|
| Durchschnittliche Wiederfindung bei N (2,5 - 100 mg/l): 91 % Durchschnittliche Wiederfindung bei C (2,5 - 100 mg/l N): 99 % | | | | | | | |

**Frisch angesetzt (4.12.):**

| **100 mg/l N:** | **50 mg/l N:** | **10 mg/l N:** | **2,5 mg/l N:** |
|---|---|---|---|
| 94,8 | 44,6* | 9,5 | 2,4 |
| 94,8 % | 89,2 %* | 95,0 % | 96,0 % |
| **428,7 mg/l C:** | **214,4 mg/l C:** | **42,9 mg/l C:** | **10,7 mg/l C:** |
| 442,6 | 218,6 | 43,7 | 10,9 |
| 103,1 % | 102,0 | 101,9 | 101,9 |

| | | | |
|---|---|---|---|
| Durchschnittliche Wiederfindung bei N (2,5 - 100 mg/l): 95 % Durchschnittliche Wiederfindung bei C (2,5 - 100 mg/l N): 102% | | | |

**Kaliumhexacyanoferant (II)**

| | **30.11** | **1,12** | **1.12** | **2.12** | **5.12** | **6.12** | **Ø** | **Ø (%)** |
|---|---|---|---|---|---|---|---|---|
| **100 mg/l N:** | 86,5 | 85,3 | 87,1 | 81,9 | 95.0 | 93,7 | **88,3** | **88,3 %** |
| **50 mg/l N:** | 44,2 | 45,0 | 44,7 | 42,4 | 47,3 | 47,7 | **45,2** | **90,4 %** |
| **10 mg/l N:** | 9,3 | 9,6 | 9,5 | 9,1 | 9,6 | 9,5 | **9,4** | **94,0 %** |
| **2,5 mg/l N:** | 2,2 | 2,5 | 2,2 | 2,3 | 2,4 | 2,3 | **2,3** | **92,0 %** |
| **85,8 mg/l C:** | 88,2 | 88,5 | 88,1 | 85,4 | 93,1 | 93,4 | **89,5** | **104,3 %** |
| **42,9 mg/l C:** | 44,5 | 44,4 | 44,7 | 42,6 | 46,9 | 47,9 | **45,2** | **105,4 %** |
| **8,6 mg/C:** | 9,1 | 9,1 | 9,2 | 9,0 | 9,3 | 9,5 | **9,2** | **107,0 % *** |
| **2,1 mg/l C:** | 2,3 | 2,8 | 2,4 | 2,1 | 2,6 | 3,0 | **2,5** | **119,0 % *** |

| | | | | | | | | |
|---|---|---|---|---|---|---|---|---|
| Durchschnittliche Wiederfindung bei N (2,5 - 100 mg/l): % Durchschnittliche Wiederfindung bei C (10- 100 mg/l N): 105 % | | | | | | | | |

**Frisch angesetzt (29.11):**

| **100 mg/l N:** | **50 mg/l N:** | **10 mg/l N:** | **2,5 mg/l N:** |
|---|---|---|---|
| 98,9 | 51,3 | 10,0 | 2,6 |
| 98,9 % | 102,6 % | 100,0 % | 104,0 % |
| **85,8 mg/l C:** | **42,9 mg/l C:** | **8,6 mg/l C:** | **2,1 mg/l C:** |
| 81,2 | 42,5 | 8,8 | * |
| 94,6 % | 99,1 % | 102,3% | * |

| | | | |
|---|---|---|---|
| Durchschnittliche Wiederfindung bei N (2,5 - 100 mg/l): 101 % Durchschnittliche Wiederfindung bei C (10 - 100 mg/l N): 99 % | | | |

Die mit * markierten Werte wurden bei den Berechnungen nicht berücksichtigt, da es sich entweder um Ausreißer handelt, oder der Messbereich ist für die Bestimmung zu klein.

## Patentansprüche

1. Katalysator für die Bestimmung der Total Carbon (TC)- und Total-Nitrogen-bound (TNb)-Werte von Wasserproben, insbesondere von Abwasserproben, umfassend ein Trägermaterial (10) und als Gemisch auf dessen Oberfläche (11) aufgebrachte Cobalt(II/III)oxide und Chrom(III)oxid (12),
**dadurch gekennzeichnet,**
**daß** das Trägermaterial körniger Bimsstein ist.

2. Katalysator nach Anspruch 1,
**dadurch gekennzeichnet,**
**daß** das Gemisch aus Cobalt(II/III)oxiden und Chrom(III)oxid (12) aufgebrannt ist.

3. Katalysator nach Anspruch 2,
**dadurch gekennzeichnet,**
**daß** auf das Trägermaterial (10) ein Gemisch von Cobaltnitrat,
Ammoniumdichromat und Wasser aufgebracht ist, das nach dem Trocknen unter Umwandlung in Cobalt(II/III)oxid und Chrom(III)oxid (12) aufgebrannt ist.

4. Katalysator nach Anspruch 2 oder 3,
**dadurch gekennzeichnet,**
**daß** die Cobalt(II/III)oxide und Chrom(III)oxid (12) als Gemisch in zwei getrennten Beschichtungsvorgängen auf das Trägermaterial (10) aufgebrannt sind.

5. Katalysator nach einem der vorhergehenden Ansprüche,
**dadurch gekennzeichnet,**
**daß** das Trägermaterial (10) aus Bimsstein eine Körnung von 0,5 - 5 mm, vorzugsweise von 0,8 - 3 mm aufweist.

6. Verwendung eines Katalysators nach einem der vorhergehenden Ansprüche zur Messung des Gehaltes an gebundenem Stickstoff in einer Probe und/oder gebundenem Kohlenstoff in einer Probe über Verbrennungsprodukte der Probe.

7. Verwendung nach Anspruch 6,
**dadurch gekennzeichnet,**
**daß** die Probe eine Wasserprobe, insbesondere eine Abwasserprobe ist.

8. Verwendung von Bimsstein als Träger für einen Katalysator in einer Vorrichtung zur Bestimmung der Total Carbon (TC) und Total-Nitrogen-bound (TNb)-Werte mit durch den Katalysator katalysierter Verbrennung von Stickstoff und/oder Kohlenstoff enthaltenden Verbindungen.

## Claims

1. A catalyst for the determination of the total carbon (TC) and total nitrogen-bound (TNb) values of water samples, particularly waste water samples, comprising a supporting material (10) and cobalt(II/III) oxides and chromium(III) oxide (12) as applied to the surface thereof as a mixture,
**characterized in that**
the supporting material is granular pumice.

2. The catalyst according to claim 1,
**characterized in that**
the mixture of cobalt(II/III) oxides and chromium(II) oxide (12) is fired thereon.

3. The catalyst according to claim 2,
**characterized in that**
the supporting material (10) has applied thereto a mixture of cobalt nitrate, ammonium dichromate, and water which when dried is fired thereon while being converted into cobalt(II/III) oxide and chromium(III) oxide (12).

4. The catalyst according to claim 2 or 3,
**characterized in that**
the cobalt(II/III) oxides and chromium(III) oxide (12) are fired, as a mixture, onto the supporting material (10) in two separate coating procedures.

5. The catalyst according to any one of the preceding claims,
**characterized in that**
the supporting material (10) of pumice has a grain size of 0.5 - 5mm, preferably 0.8 - 3mm.

6. A use of a catalyst according to any one of the preceding claims for measuring the content of bound nitrogen in a sample and/or bound carbon in a sample via the combustion products of the sample.

7. The use according to claim 6,
**characterized in that**
the sample is a water sample, particularly a waste water sample.

8. A use of pumice as a supporting material for a catalyst in a device for the determination of the total carbon (TC) and total nitrogen-bound (TNb) values under a combustion of nitrogen-containing and/or carbon-containing compounds, which is catalyzed by the catalyst.

## Revendications

1. Catalyseur pour la détermination des valeurs de carbone total (TC) et d'azote lié total (TNb) d'échantillons d'eau, en particulier d'échantillons d'eaux usées, comprenant un support (10) ainsi que des oxydes de cobalt (II/III) et de l'oxyde de chrome (III) (12) appliqués comme mélange sur la surface (11) de ce support,
**caractérisé par le fait**
**que** ledit support est de la pierre ponce granuleuse.

2. Catalyseur selon la revendication 1,
**caractérisé par le fait**
**que** ledit mélange en oxydes de cobalt (II/III) et en oxyde de chrome (III) (12) est appliqué par cuisson.

3. Catalyseur selon la revendication 2,
**caractérisé par le fait**
**qu'**un mélange de nitrate de cobalt, de dichromate d'ammonium et d'eau est appliqué sur le support (10), ledit mélange étant appliqué par cuisson après le séchage tout en le transformant en oxyde de cobalt (II/III) et en oxyde de chrome (III) (12).

4. Catalyseur selon la revendication 2 ou 3,
**caractérisé par le fait**
**que** les oxydes de cobalt (II/III) et l'oxyde de chrome (III) (12) sont appliqués par cuisson en tant que mélange en deux opérations séparées de revêtement sur ledit support (10).

5. Catalyseur selon l'une des revendications précédentes,
**caractérisé par le fait**
**que** ledit support (10) en pierre ponce présente une granulométrie comprise entre 0,5 et 5 mm, de préférence entre 0,8 et 3 mm.

6. Mise en oeuvre d'un catalyseur selon l'une des revendications précédentes, pour la mesure de la teneur en azote lié dans un échantillon et/ou en carbone combiné dans un échantillon par l'intermédiaire de produits de combustion de l'échantillon.

7. Mise en oeuvre selon la revendication 6,
**caractérisée par le fait**
**que** ledit échantillon est un échantillon d'eau, en particulier en échantillon d'eaux usées.

8. Utilisation de pierre ponce en tant que support pour un catalyseur dans un dispositif pour la détermination des valeurs de carbone total (TC) et d'azote lié total (TNb) avec une combustion catalysée par le catalyseur, de composés contenant de l'azote et/ou du carbone.
